# EUROPEAN PATENT SPECIFICATION

(11) **EP 1 338 332 B1**
(45) Date of publication and mention of the grant of the patent: **11.08.2004**
(21) Application number: 03003170.2
(22) Date of filing: 19.02.2003
(51) Int. Cl.: B01F 13/00

(54) **Kneading apparatus and kneading method**
Knetvorrichtung und Verfahren zum Kneten
Appareil de pétrissage et procédé pour le pétrissage

(30) Priority: 20.02.2002 JP 2002042637
(43) Date of publication of application: 27.08.2003
(73) Proprietor: MITSUBISHI MATERIALS CORPORATION, Chiyoda-ku, Tokyo (JP)
(72) Inventor: Takeuchi, Hiroyasu, c/o Mitsubishi Materials Corp., Chiyoda-ku, Tokyo (JP); Hirano, Masahiro, c/o Mitsubishi Materials Corp., Yokozemachi, Chichibu-gun, Saitama-ken (JP); Asaoka, Nobuyuki, c/o Mitsubishi Materials Corp., Yokozemachi, Chichibu-gun, Saitama-ken (JP)
(74) Representative: Gille Hrabal Struck Neidlein Prop Roos

(56) References cited:
- WO-A-01/47571
- WO-A-98/57734
- GB-A- 1 340 483
- US-A- 4 743 229

## Description

### BACKGROUND OF THE INVENTION

### Field of the Invention

This invention relates to a kneading apparatus and method, which are suitable for kneading objects.

### Description of Related Art

A kneading apparatus of this type has been disclosed in, for example, Japanese Laid-Open Patent Application No. 5-103966. This kneading apparatus comprises two chambers which are provided at the left and right ends of a narrow tube, the capacities of the chambers being varied by pistons, and an injection / discharge hole for a liquid (object of kneading), which is connected at the top of the narrow tube and is isolated from the outside by a rubber-like lid.

In the kneading apparatus described above, the left and right pistons move back and forth along the same direction, thereby conveying the liquid along the narrow tube; the liquid is kneaded by the turbulence generated at this time.

However, in this kneading apparatus, since the injection / discharge hole is provided in the top of the narrow tube, liquid which has accumulated in the injection /discharge hole does not flow easily into the narrow tube, and the injected liquid is not sufficiently kneaded. Since it becomes necessary to use an injector to inject the liquid into the injection / discharge hole, and to extract the liquid from the injection / discharge hole, the operation of injecting and extracting the liquid becomes troublesome. Further, it is necessary to hold the cylinder section comprising the two chambers and the narrow tube by hand, and push one of the pistons into it, making the kneading operation troublesome and increasing the probability of infiltration by bacteria and the like.

### SUMMARY OF THE INVENTION

This invention has been realized in consideration of the problems mentioned above, and aims to provide a kneading apparatus and method capable of sufficiently kneading an object, and simplifying the insertion and extraction of the object before and after kneading, with a low probability of infiltration of bacteria and the like.

In order to achieve the above objects, the kneading apparatus according to a first aspect of this invention comprises a first cylinder having a first chamber, the capacity of the first chamber being varied by the motion of a first piston, and a first narrow hole, which is provided in a tip of the first cylinder and connects to the first chamber; a second cylinder having a second chamber, the capacity of the second chamber being varied by the motion of a second piston, and a second narrow hole, which is provided in a tip of the second cylinder and connects to the second chamber. The first and second cylinders are arranged on the axial line with their tips separably joined together, so that the first and second chambers are connected together by at least one of the first narrow hole and the second narrow hole. The kneading apparatus also comprises a cylinder support unit, which holds the base of the first piston which protrudes from the base of the first cylinder, and the base of the second piston which protrudes from the base of the second cylinder, in such a manner that they can be freely attached and removed. An object of kneading is inserted into the first chamber and/or the second chamber, and is kneaded by the motion of the first and second cylinders in the axial direction.

According to the present invention, the object of kneading can be sufficiently kneaded by moving the first and second cylinders. There is no need to hold the first and second pistons by hand, reducing the area touched by hand and thereby reducing the probability of infiltration of bacteria. Further, since the pistons do not necessarily have to be moved in the push direction, the kneading operation is simplified.

The object of kneading can be easily inserted into the first chamber and the second chamber, for example, by drawing it through the tips, or by removing the first piston and the second piston. After the kneading operation has ended, the object of kneading can be easily removed from the first and second chambers by pushing first and second pistons. There is a further advantage in that, since the cement powder and the hardening liquid are kneaded in an airtight space sealed by the first narrow hole and/or the second narrow hole, the first chamber, and the second chamber, the amount of air entering the object of kneading is extremely small.

According to a second aspect, in the kneading apparatus of the first aspect, the object of kneading comprises a cement powder and a hardening liquid which reacts with the cement powder. In this case, it is possible to easily achieve the cement powder and the hardening liquid which have been sufficiently kneaded and just start to harden. Since the amount of air entering the object of kneading is extremely small, after hardening, the cement material is extremely strong.

According to a third aspect, in the kneading apparatus of the first and second aspects, an injection unit can be attached to the tip(s) of the first cylinder and/or the second cylinder. In this case, the object of kneading can be easily injected into an object of injecting by attaching an injection unit to, for instance, the tip of the first cylinder. This offers an advantage that, when the cement powder and the hardening liquid have been kneaded and just start to harden, they can be immediately injected into, for example, a bone which is full of pores caused by osteoporosis.

According to a fourth aspect, the kneading method of this invention comprises the steps of arranging a first cylinder having a first chamber, the capacity of the first chamber being varied by the motion of a first piston, and a first narrow hole, which is provided in a tip of the first cylinder and connects to the first chamber, and a second cylinder having a second chamber, the capacity of the second chamber being varied by the motion of a second piston, and a second narrow hole, which is provided in a tip of the second cylinder and connects to the second chamber, on an axial line with their tips separably joined together, so that the first and second chambers are connected together by at least one of the first narrow hole and the second narrow hole; and using a cylinder support unit to hold the base of the first piston which protrudes from the base of the first cylinder, and the base of the second piston which protrudes from the base of the second cylinder, in such a manner that they can be freely attached and removed, while kneading an object of kneading, which has been inserted into the first chamber and/or the second chamber, by the motion of the first and second cylinders in the axial direction.

According to a fifth aspect, in the kneading method of the fourth aspect, the object of kneading comprises a cement powder and a hardening liquid which reacts with the cement powder.

According to a sixth aspect, in the kneading apparatus of the fourth and fifth aspects, after the object of kneading has been kneaded, an injection unit is attached to the tip(s) of the first cylinder and/or the second cylinder, and the object of kneading is injected into an object of injecting.

According to the present invention, after the connection between the tips of the first cylinder and the second cylinder has been disengaged, the object of kneading is drawn from the tips by pulling at least one of the pistons. Consequently, the object of kneading is inserted into the first chamber and/or the second chamber. Then, after the tips of the first cylinder and the second cylinder have been connected together, the cylinder support unit holds the base of the first piston and the base of the second piston. This completes the preparation for kneading. Alternatively, the object of kneading may be inserted into the first chamber and/or the second chamber by other methods such as pulling the piston out from the cylinder.

Holding the cylinder support unit in the hand, the first cylinder and/or the second cylinder is/are moved in the axial direction. The first and second cylinders move together, and the object of kneading which has for example been inserted into the first chamber passes through the first narrow hole and/or the second narrow hole to the second chamber; alternatively, the object of kneading which has been inserted into the second chamber passes through the first narrow hole and/or the second narrow hole into the first chamber.

Turbulence, differences in current speeds, and the like, are generated by squeezing as the object of kneading flows through the first and second narrow holes, by the rapid movement through the first narrow hole and the like, and by spillage from the first narrow hole and the like to the first and second chambers; in a case where two or more substances have been inserted for kneading, these substances are kneaded very speedily. Therefore, by repeating the backward and forward motion of the first cylinder and the second cylinder, the object of kneading can be sufficiently kneaded.

Moreover, at the time of kneading, it is sufficient to hold at least one of the first cylinder and the second cylinder in the hand and move it in the axial direction, it being unnecessary to hold the first and second pistons in the hand, thereby reducing the area touched by hand. This reduces the probability of infiltration by bacteria. Since the pistons do not necessarily have to be moved in the push direction, the kneading operation is simplified.

After the kneading operation, the object of kneading can be easily removed from the tips of the first and second cylinders by removing them from the cylinder support unit and separating their tips. In other words, the object of kneading remaining in the first and second chambers can be easily removing from the tips simply by pushing the first and second pistons. This simplifies the insertion and removal of the object of kneading. There is a further advantage in that, since the kneading is performed in an airtight space sealed by the first narrow hole and/or the second narrow hole, the first chamber, and the second chamber, the amount of air entering the object of kneading is extremely small.

In the second and fifth aspects of the invention, the cement powder is for example pouring into the first chamber from the tip of the first cylinder using a funnel after disconnecting the tips of the cylinders from each other, and a hardening liquid is drawn into the second chamber from the tip of the second cylinder by pulling the second piston; thereafter, the tips are connected together, and the first and second cylinders are moved after attaching the cylinder support unit thereto, thereby kneading the cement powder and the hardening liquid sufficiently, until they just start to harden. Moreover, since the amount of air entering the object of kneading is extremely small, after hardening, the hardened cement material is extremely strong.

Alternatively, the cement powder and the hardening liquid may be inserted into, for example, one of the first chamber and second chamber, and kneaded by moving the first and second cylinders.

When a cement powder and a hardening liquid are used as the object of kneading, the inner diameter of the first and second narrow holes should preferably be between 2 mm and 10 mm. When the inner diameter is less than 2 mm, the motion resistance of the cement powder is particular too strong, preventing the first and second cylinders from moving smoothly; when the inner diameter exceeds 10 mm, the squeeze effect is reduced, making it impossible to sufficiently mix and knead the cement powder and the hardening liquid within a short period of time, and reducing the strength of the cement after it has hardened.

When D1 and D2 respectively represent the inner diameters of the first and second cylinders, and d1 and d2 respectively represent the inner diameters of the first and second narrow holes, the contraction rate for the diameters of the narrow holes, which are calculated using the ratio of d1/D1, d2/D2, d1/D2, and d2/D1, are preferably between 10% and 50%. The reason for this is that when the contraction rate is less than 10%, the squeeze factor increases and the motion resistance of the cement powder and the like becomes excessive; when the contraction rate is more than 50%, the squeeze effect is reduced, making it impossible to sufficiently mix and knead the cement powder and the like within a short period of time. A contraction rate for the diameter of the narrow hole of between 10% and 50% enables the apparatus to be applied to objects of kneading other than one comprising a combination of cement powder and hardening liquid.

According to the third and sixth aspects of this invention, the object of kneading can be easily injected into an object of injecting by attaching an injection unit to, for instance, the tip of the first cylinder. For example, when the cement powder and the hardening liquid have been kneaded and they just start to harden, they can be immediately injected into, for example, a bone (object of injecting) which is full of pores caused by osteoporosis.

### BRIEF DESCRIPTION OF THE DRAWINGS

Fig. 1 is a cross-sectional view of a kneading apparatus according to a first embodiment of this invention;
Fig. 2 is a diagram of the same kneading apparatus, being a cross-sectional view taken along the line II - II of Fig. 1;
Fig. 3 is a diagram of the same kneading apparatus, being a cross-sectional view taken along the line III - III of Fig. 1;
Fig. 4 is a cross-sectional view showing primary parts of the same kneading apparatus;
Fig. 5 is a cross-sectional view showing primary parts of a kneading apparatus according to a second embodiment of this invention;
Fig. 6 is a cross-sectional view showing primary parts of a kneading apparatus according to a third embodiment of this invention;
Fig. 7 is a cross-sectional view showing primary parts of a kneading apparatus according to a fourth embodiment of this invention; and
Fig. 8 is a cross-sectional view showing primary parts of a kneading apparatus according to a fifth embodiment of this invention.

### DESCRIPTION OF THE PREFERRED EMBODIMENTS

Preferred embodiments of the invention will be explained with reference to the drawings.

### Embodiment 1

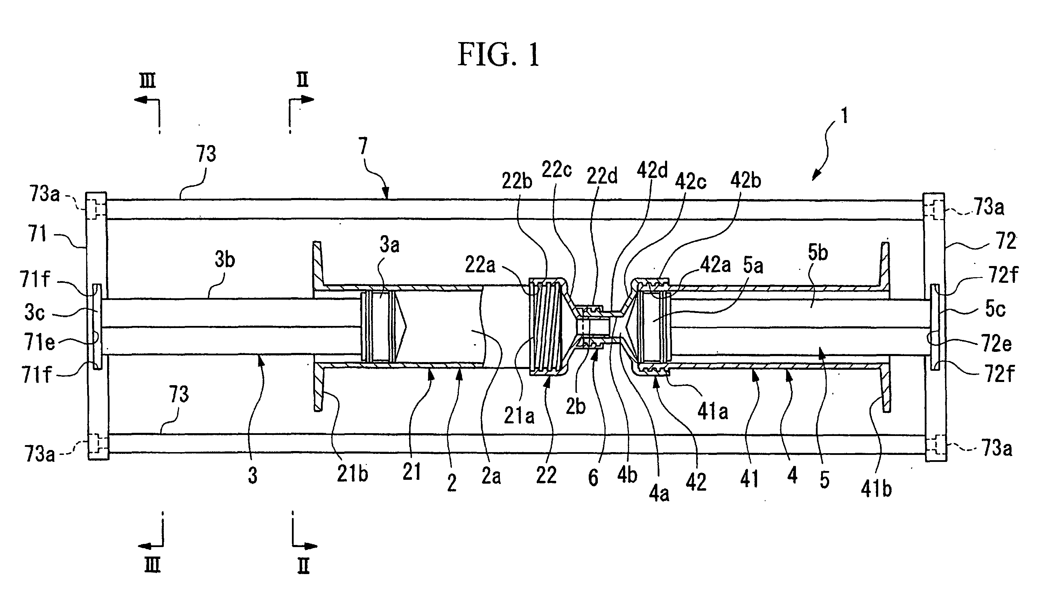

Figs. 1 to 4 show a first embodiment of this invention. The kneading apparatus 1 of the first embodiment comprises a first cylinder 2 and a second cylinder 4. The first cylinder 2 has a first chamber 2a having a capacity which is varied by the movement of a first piston 3, and a first narrow hole 2b leading to the first chamber 2a in its tip 22d. The second cylinder 4 has a second chamber 4a having a capacity which is varied by the movement of a second piston 5, and a second narrow hole 4b leading to the second chamber 4a in its tip 42d.

The first cylinder 2 and second cylinder 4 are provided along a horizontal line with their tips 22d and 42d separably connected by a thread mechanism 6; thus connected, the first chamber 2a and the second chamber 4a are joined together via the first narrow hole 2b and the second narrow hole 4b.

The first cylinder 2 and the second cylinder 4 respectively comprise cylindrical sections 21 and 41, and head sections 22 and 42 which are connected by screws at the tips of the cylindrical sections 21 and 41 respectively.

The cylindrical sections 21 and 41 have male screws 21a and 41a around the outer rims of their tips, and flanges 21b and 41b, which extend outwardly in a direction parallel to the radius from their respective base sections.

The head sections 22 and 42 comprise in one body (a) large-diameter cylindrical sections 22b and 42b having in their inner faces female screws 22a and 42a respectively, which screw together with male screws 21a and 41a, (b) tapered sections 22c and 42c which become narrower as they lead from the large-diameter cylindrical sections 22b and 42b toward the tips, and (c) tip sections 22d and 42d, provided at the tip end of the tapered sections 22c and 42c. Sealing members, such as O-rings, may be inserted between the tip positions of the sections where the male screws 21a and 41a are provided in the cylindrical sections 21 and 41, and the deepest points of the sections where the female screws 22a and 42a are provided in the head sections 22 and 42.

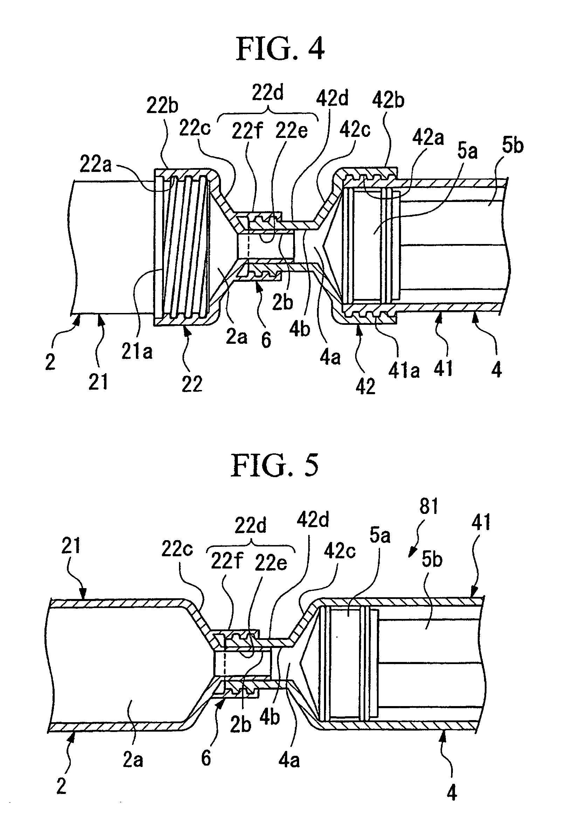

As shown in Fig. 4, the tip section 22d of the first cylinder 2 is comprised of an inner circular pipe 22e and an outer circular pipe 22f, which are provided around the same axis, and the tip section 42d of the second cylinder 4 comprises a circular pipe which is small enough to fit between the inner circular pipe 22e and the outer circular pipe 22f.

The thread mechanism 6 comprises a female screw in the inner peripheral face of the outer circular pipe 22f in one tip section 22d, and a male screw in the outer peripheral face of the other tip section 42d, which screws into the female screw. The tip sections 22d and 42d are kept sealed from the outside by connecting the outer peripheral face of the inner circular pipe 22e to the inner peripheral face of the tip section 42d, or by inserting a viscous sealant into the connection.

Furthermore, the tip 22d can be held in place by clipping the outer periphery of the inner circular pipe 22e into the base section of an unillustrated injection needle (injection means). The other tip section 42d can be held by clipping its outer periphery into the base section of an injection needle.

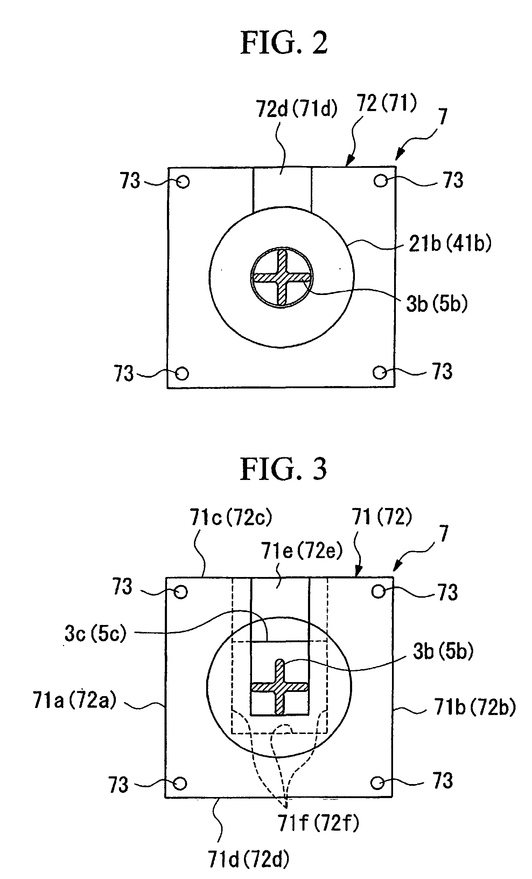

As shown in Fig. 1, the first piston 3 and the second piston 5 respectively comprise piston heads 3a and 5a, and piston rods 3b and 5b which extend from the piston heads 3a and 5a toward the bases of the cylinders 2 and 4, and protrude from the bases (the positions of the flanges 21b and 41b). As shown in Fig. 3, the piston rods 3b and 5b are cross-shaped in cross-section. The bases of the piston rods 3b and 5b comprise square-shaped guiding plates 3c and 5c, which protrude at right angles to the axial direction. It is preferable that these guiding plates 3c and 5c have square shapes, in consideration of the moldability with resins, and in consideration of the easiness of insertion of the guiding plates 3c and 5c into the clip grooves 71f and 72f. However, the guiding plates 3c and 5c may have other shapes such as rectangular shapes.

The piston rods 3b and 5b may be round, square, circular, or multi-sided, in cross-section.

The first cylinder 2, the second cylinder 4, the first piston 3, and the second piston 5 comprise single pieces of compound resin such as PP (polypropylene) or PE (polyethylene), and are discarded after a single kneading operation.

The first cylinder 2 and second cylinder 4 are connected together by the thread mechanism 6, but can be freely attached and removed by a cylinder support unit 7, provided via guide plates 3c and 5c of the first piston 3 and second piston 5.

As shown in Fig. 1, the cylinder support unit 7 comprises a first supporting member 71 which holds the guide plate 3c of the first piston 3 so that it can be freely attached and removed, a second supporting member 72 which holds the guide plate 5c of the second piston 5 so that it can be freely attached and removed, and four supporting bars 73 which hold the first supporting member 71 and the second supporting member 72 parallel to each other with a predetermined interval therebetween.

As shown in Fig. 3, in the present embodiment, the first supporting member 71 and the second supporting member 72 comprise square plates of a metal such as stainless steel, brass, or the like, the left edges 71a and 72a being provided parallel to the right edges 71b and 72b, and the top edges 71c and 72c being provided parallel to the bottom edges 71d and 72d respectively. Therefore, it is possible to place the first supporting member 71 and the second supporting member 72, for example, on a horizontal table, so as to support the first and second cylinders 2 and 4 in a horizontal state or in a vertical state. Thus, an advantage can be obtained in that cement powder and a hardening liquid are stably maintained in the cylinders for a moment after or before mixing them. The shapes of the supporting members 71 and 72 may be changed to other shapes.

Furthermore, guide grooves 71e and 72e are provided along the center lines from the left to the right of each of the top edges 71c and 72c on one face of the first supporting member 71 and the second supporting member 72 respectively, and extend as far as the central section of the first and second supporting members 71 and 72. Clip grooves 71 f and 72f are provided in the guide grooves 71e and 72e, and guide the side edges of the guide plates 3c and 5c in a freely movable manner while preventing them from becoming detached at right angles to the first and second supporting members 71 and 72.

The supporting bars 73 comprise round-headed rods of a metal such as stainless steel or bronze, and their end sections are secured at the four corners of the first and second supporting members 71 and 72 by bolts 73a (see Fig. 1).

The above metal makes the cylinder support unit 7 highly rigid, and suitable for repeated use in kneading.

Alternatively, the first and second supporting members 71 and 72, and the supporting bars 73 and the like, of the cylinder support unit 7 may be comprised of a material other than metal, such as a hard and strong plastic. This achieves a highly rigid cylinder support unit 7.

In the kneading apparatus 1 of the constitution described above, the cylinders 2 and 4 comprising the pistons 3 and 5 are firstly removed from the cylinder support unit 7, the thread mechanism 6 is disengaged, and the tips 22d and 42d are isolated. Then, for example, the head section 22 is detached from the first cylinder 2, and cement powder (object of kneading) is inserted into the first chamber 2a. In addition, the second piston 5 of the second cylinder 4 is pulled so as to draw a hardening liquid (object of kneading) through the second narrow hole 4b of the tip 42d into the second chamber 4a. Alternatively, the first piston 3 may be pulled out from the first cylinder 2, and the cement powder may be inserted to the first cylinder 2. The hardening liquid may be inserted into the second cylinder 4 after detaching the head section 42 or pulling the second piston 5. The cement powder may be inserted into the second cylinder 4, and the hardening liquid may be inserted into the first cylinder 2. After inserting the cement powder and the hardening liquid into the cylinders 2 and 4, the head section 22 and 42 and the pistons 3 and 5 are attached respectively to the original positions. Furthermore, the tips 22d and 42d are joined together, and the guide plates 3c and 5c of the first piston 3 and the second piston 5 are inserted into the guide grooves 71e and 72e of the first supporting member 71 and the second supporting member 72 respectively. This attaches the cylinders 2 and 4 comprising the pistons 3 and 5 to the cylinder support unit 7, and completes the preparation for kneading the cement powder and the hardening liquid.

The supporting bars 73 of the cylinder support unit 7 are placed in the palm of the hand, while holding at least one of the first cylinder 2 and the second cylinder 4 and moving it in the axial direction. Consequently, the first cylinder 2 and the second cylinder 4 move together in the axial direction, so that, for instance, the cement powder in the first chamber 2a passes through the first narrow hole 2b and the second narrow hole 4b into the second chamber 4a containing the hardening liquid; this motion is repeated backward and forward, so that the cement powder and the hardening liquid passes through the first narrow hole 2b and the second narrow hole 4b and move between the first chamber 2a and the second chamber 4a.

At this time, turbulence, differences in current speeds, and the like, are generated by the flow into the first narrow hole 2b and the second narrow hole 4b, by the rapid movement of the first narrow hole 2b and the second narrow hole 4b, and by spillage from the first narrow hole 2b and second narrow hole 4b to the second chamber 4a and first chamber 2a, and accelerate the kneading of the cement powder and the hardening liquid.

When the object of kneading (i.e. the cement powder and the hardening liquid) just starts to harden, the first cylinder 2 and the second cylinder 4 are removed from the cylinder support unit 7, the connection between the tips 22d and 42d is disengaged, and an injection needle is attached to, for instance, the tip 22d of the first cylinder 2 on the side which is filled with the object of kneading. The object of kneading is injected into, for instance, a bone (object of injecting) which is full of pores caused by osteoporosis.

Since the cement powder and the hardening liquid are kneaded in an airtight space sealed by the first chamber 2a, the second chamber 4a, the first narrow hole 2b, and the second narrow hole 4b, the amount of air entering the object of kneading is extremely small. Therefore, after hardening, the cement material is extremely strong.

In the case where the head section 22 and 42 are detached or the pistons 3 and 5 are pulled out in order to insert the cement powder and the hardening liquid into the cylinders 2 and 4, the tip 22d and the tip 42d may remain connected.

Alternatively, kneading can be performed by moving the first cylinder 2 and the second cylinder 4 while, for instance, one of the first and second chambers 2a and 4a is filled with the cement powder and the hardening liquid.

When cement powder and the hardening liquid is used as the object of kneading, as in the case described above, the inner diameter of the first narrow hole 2b on the inner side should preferably be between 2 mm and 10 mm. When the inner diameter is less than 2 mm, the motion resistance of the cement powder is particular is too strong, preventing the first cylinder 2 and the second cylinder 4 from moving smoothly; when the inner diameter exceeds 10 mm, the squeeze effect is reduced, making it impossible to sufficiently mix and knead the cement powder and the hardening liquid within a short period of time, and reducing the strength of the cement after it has hardened. Incidentally, the inner diameter of 2 mm to 10 mm mentioned above is particularly effective when the inner diameters of the first cylinder 2 and the second cylinder 4 are between 5 mm and 30 mm.

When D1 and D2 respectively represent the inner diameters of the first and second cylinders 2 and 4, and d1 and d2 respectively represent the inner diameters of the first and second narrow holes 2b and 4b, the contraction rates for the diameter of the narrow hole, which are calculated using the ratio of d1/D1, d2/D2, d1/D2, and d2/D1, are preferably between 10% and 50%. The reason for this is that when the contraction rate is less than 10%, the squeeze factor increases and the motion resistance of the cement powder and the like becomes excessive; when the contraction rate is more than 50%, the squeeze effect is reduced, making it impossible to sufficiently mix and knead the cement powder and the like within a short period of time. A contraction rate for the diameter of the narrow hole of between 10% and 50% enables the apparatus to be applied to objects of kneading other than one comprising a combination of cement powder and hardening liquid.

The cement powder may be, for instance, cement having main components of alpha tricalcium phosphate, tetracalcium phosphate, and the like. More specifically, it may be alpha tricalcium phosphate or tetracalcium phosphate as simple substances or a mixture of the two, a mixture of alpha tricalcium phosphate and dicalcium phosphate and/or monocalcium phosphate, a mixture of alpha tetracalcium phosphate and dicalcium phosphate and/or monocalcium phosphate, a mixture of alpha tricalcium phosphate, tetracalcium phosphate, dicalcium phosphate and/or monocalcium phosphate, and the like.

The hardening liquid should preferably comprise water, polyacrylic acid, citric acid, malic acid, or an aqueous solution made by dissolving an organic acid of a mixture of these, sodium chloride, sodium chondroitin sulphate, sodium succinate, sodium lactate, or an aqueous solution made by dissolving aqueous salts of a mixture of these, and the like.

As described above, according to the kneading apparatus 1 of this embodiment, the object of kneading can be sufficiently kneaded. Moreover, at the time of kneading, it is sufficient to hold at least one of the first cylinder 2 and the second cylinder 4 in the hand and move it in the axial direction, it being unnecessary to hold the first piston 3 and the second piston 5 in the hand, reducing the area touched by hand. This reduces the probability of infiltration by bacteria. Since the pistons do not necessarily have to be moved in the push direction, the kneading operation is simplified.

The object of kneading can be easily inserted into the first chamber 2a and the second chamber 4a by removing and attaching the first piston 3 and the second piston 5, and, after the kneading operation, the object of kneading can be easily removed from the first chamber 2a and the second chamber 4a by pushing the first piston 3 and the second piston 5.

### Embodiment 2

Subsequently, a second embodiment of this invention will be explained based on Fig. 5. Parts which are the same as those in the first embodiment are represented by the same reference codes and are not described further.

In the kneading apparatus 81 of the second embodiment, the head sections 22 and 42 shown in the first embodiment are provided in a single piece with the cylindrical section 21 and cylindrical section 41 respectively. The tip 22d comprises the tapered section 22c, the inner circular pipe 22e, and the outer circular pipe 22f, and is provided in a single piece with the cylindrical section 21 of the first cylinder 2. The tapered section 42c and the tip 42d are provided in a single piece with the cylindrical section 41 of the second cylinder 4.

In the kneading apparatus 81, the hardening liquid can be inserted into the first chamber 2a or the second chamber 4a by drawing it in from the tips 22d and 42d. With regard to the cement powder, it can be inserted into the first chamber 2a or the second chamber 4a by pouring the cement powder from the separated tips 22d and 42d using a funnel, or by removing the first piston 3 and the second piston 5.

### Embodiment 3

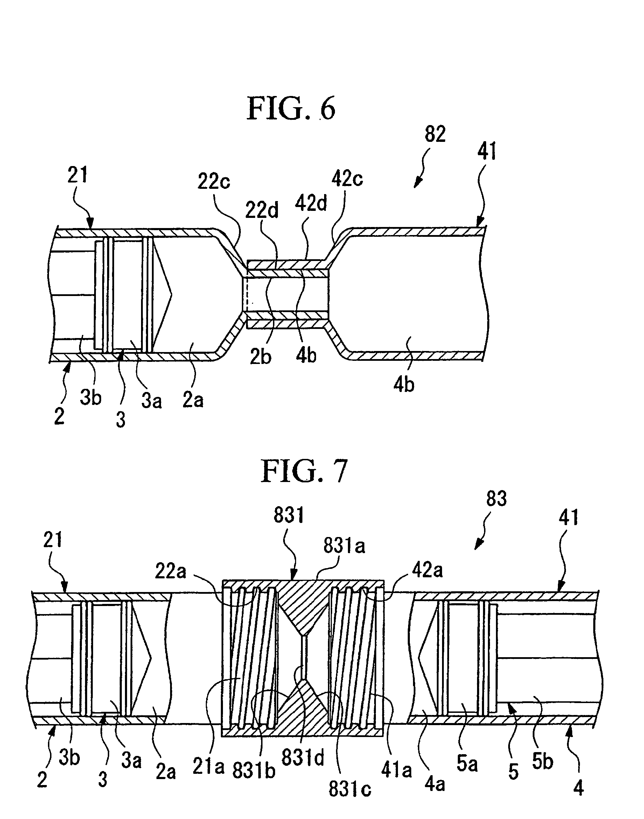

Subsequently, a third embodiment of this invention will be explained based on Fig. 6. Parts which are the same as those in the second embodiment are represented by the same reference codes and are not described further.

In the constitution of the kneading apparatus 82 of the third embodiment, the tip 22d shown in the second embodiment comprises a single circular pipe, and the other tip 42d fits around the outer peripheral face of the tip 22d. The connection between the inner and outer peripheral faces of the two tips 22d and 42d, into which a viscous sealant may also be inserted, keeps them airtight from the outside.

The cement powder and the hardening liquid pass only through the first narrow hole 2b on the inner side.

In the kneading apparatus 82, the cement powder and the hardening liquid are inserted into the first chamber 2a and the second chamber 4a by drawing them in from the tips 22d and 42d, or by removing and attaching the first piston 3 and the second piston 5.

### Embodiment 4

Subsequently, a fourth embodiment of this invention will be explained based on Fig. 7. Parts which are the same as those in the first embodiment are represented by the same reference codes and are not described further.

Instead of the head sections 22 and 42 shown in the first embodiment, the kneading apparatus 83 of the fourth embodiment comprises a joint unit 831 in which the head sections 22 and 42 are connected in a single piece.

That is, the joint unit 831 comprises female screws 22a and 42a, tapered faces 831b and 831c, and a narrow hole 831d, provided around the axis of a cylindrical main body 831a.

The female screws 22a and 42a are the same as those provided in the head sections 22 and 42 of the first embodiment, and extend to a predetermined depth in each end of the main body 831a. The tapered faces 831b and 831c become narrower as they approach each other from the female screws 22a and 42a. These tapered faces 831b and 831c correspond to the tapered sections 22c and 42c of the first embodiment.

The narrow hole 831d connects the sections where the diameters of the tapered faces 831b and 831c are at their minimum, and corresponds to the first narrow hole 2b and the second narrow hole 4b of the first embodiment. That is, the section of the narrow hole 831d on the first cylinder 2 side corresponds to the first narrow hole 2b, and the section of the narrow hole 831d on the second cylinder 4 side corresponds to the second narrow hole 4b. The joint unit 831 comprises a single piece of resin such as PP or PE.

Sealing members, such as O-rings, may be inserted between the tip positions of the sections where the male screws 21a and 41a are provided in the cylindrical sections 21 and 41, and the deepest points of the sections where the female screws 22a and 42a are provided in the joint unit 831.

In the kneading apparatus 83, the cement powder and the hardening liquid are inserted into the first chamber 2a and the second chamber 4a by removing and attaching the joint unit 831, or by removing the first piston 3 and the second piston 5.

### Embodiment 5

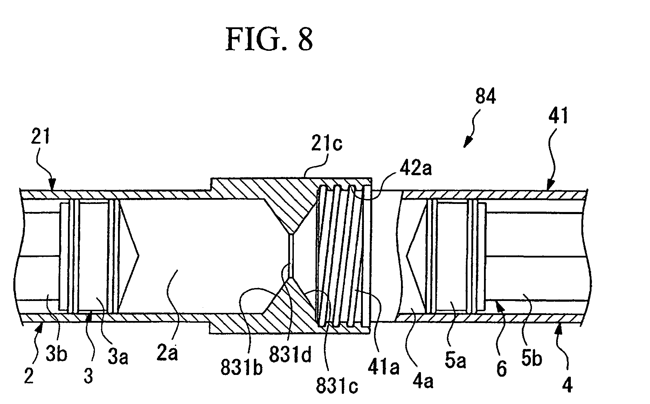

Subsequently, a fifth embodiment of this invention will be explained based on Fig. 8. Parts which are the same as those in the fourth embodiment are represented by the same reference codes and are not described further.

The kneading apparatus 84 of the fifth embodiment comprises a joint section 21c, corresponding to the joint unit 831 shown in the fourth embodiment, provided in a single piece with the cylindrical section 21 of the first cylinder 2.

The joint section 21c is provided coaxially around the tip of the cylindrical section 21, and a tapered face 831b, a narrow hole 831d, a tapered face 831c, and a female screw 42a, are provided along its inner face leading toward its tip side.

The tapered face 831b becomes gradually narrower as it approaches the tip side from the inner face of the cylindrical section 21. The narrow hole 831d, the tapered face 831c, and the female screw 42a are the same as those shown in the fourth embodiment, and will not be explained further.

Incidentally, sealing members, such as O-rings, may be inserted between the tip of the section where the male screw 41a is provided in the cylindrical section 41, and the deepest point of the section where the female screw 42a is provided in the joint section 21c.

In the kneading apparatus 84, the cement powder and/or the hardening liquid are inserted into the second chamber 4a from the tip side of the cylindrical section 41 after separating the continuation unit 21c and the cylindrical section 41; alternatively, the cement powder and/or the hardening liquid may be inserted into the second chamber 4a by pouring them through the narrow hole 831d using a funnel or by inserting them from the base side of the cylindrical section 41 after pulling out the first piston 3.

The above constitution enables the number of components to be reduced.

The cement powder may be inserted into the cylinders by drawing the cement powder through the tip 22d or 42d by pulling the pistons 3 and 5.

## Claims

1. A kneading apparatus comprising:
a first cylinder (2) having a first chamber (2a) and a first narrow hole (2b) which is provided in a tip (22d) of the first cylinder (2) and communicates with the first chamber (2a);
a first piston (3) which is movable in the first cylinder (2) to vary the capacity of the first chamber (2a);
a second cylinder (4) having a second chamber (4a) and a second narrow hole (4b) which is provided in a tip (42d) of the second cylinder (4) and communicates with the second chamber (4a);
a second piston (5) which is movable in the second cylinder (4) to vary the capacity of the second chamber (4a); and
a cylinder support unit (7) which holds a base of the first piston (3) which protrudes from the first cylinder (2), and a base of the second piston (5) which protrudes from the second cylinder (4), in such a manner that the bases can be attached and removed;
wherein the first and second cylinders (2, 4) is arranged along an axial line with their tips (22d, 42d) separably joined together, so that the first and second chambers (2a, 4a) are communicated together by at least one of the first narrow hole (2b) and the second narrow hole (4b); and whereby
an object of kneading, which may be inserted into at least one of the first chamber (2a) and the second chamber (4a), can be kneaded by the motion of the first and second cylinders (2, 4) in a direction along the axial line.

2. A kneading apparatus according to Claim 1, wherein the object of kneading comprises a cement powder and a hardening liquid which reacts with the cement powder.

3. A kneading apparatus according to Claims 1 and 2, wherein an injection unit can be attached to at least one of the tips (22d, 42d) of the first cylinder (2) and the second cylinder (4).

4. A kneading method comprising the steps of:
providing a kneading apparatus which comprises: a first cylinder (2) having a first chamber (2a) and a first narrow hole (2b) which is provided in a tip (22d) of the first cylinder (2) and communicates with the first chamber (2a); a first piston (3) which is movable in the first cylinder (2) to vary the capacity of the first chamber (2a); a second cylinder (4) having a second chamber (4a) and a second narrow hole (4b) which is provided in a tip (42d) of the second cylinder (4) and communicates with the second chamber (4a); a second piston (5) which is movable in the second cylinder (4) to vary the capacity of the second chamber (4a); and a cylinder support unit (7);
inserting an object of kneading into at least one of the first chamber (2a) and the second chamber (4a),
arranging the first cylinder (2) and a second cylinder (4) along an axial line with the tips (22d, 42d) separably joined together, so that the first and second chambers (2a, 4a) are connected together via at least one of the first narrow hole (2b) and the second narrow hole (4b);
connecting a base of the first piston (3) which protrudes from the first cylinder (2) and a base of the second piston (5) which protrudes from the second cylinder (4) to the cylinder support unit (7); and
moving the first and second cylinders (2, 4) in a direction along the axial line with respect to the cylinder support unit (7) to knead the object which has been inserted into at least one of the first chamber (2) and the second chamber (4).

5. A kneading method according to Claim 4, wherein the object of kneading comprises a cement powder and a hardening liquid which reacts with the cement powder.

6. A kneading apparatus according to Claims 4 or 5, wherein, after the object of kneading has been kneaded, an injection unit is attached to at least one of the tips (22d, 42d) of the first cylinder (2) and the second cylinder (4), and the object of kneading is injected into an object of injecting.

## Patentansprüche

1. Knetvorrichtung, die folgendes enthält:
einen ersten Zylinder (2) mit einer ersten Kammer (2a) und einer ersten engen Bohrung (2b), die in dem Kopf (22d) des ersten Zylinders (2) angeordnet ist und mit der ersten Kammer (2a) kommuniziert;
einen ersten Kolben (3), der in dem ersten Zylinder (2) verschoben werden kann, um die Kapazität der ersten Kammer (2a) zu verändern;
einen zweiten Zylinder (4) mit einer zweiten Kammer (4a) und einer zweiten engen Bohrung (4b), die in dem Kopf (42d) des zweiten Zylinders (4) angeordnet sind und
mit der zweiten Kammer (4a) kommunizieren;
einen zweiten Zylinder (5), der in dem zweiten Zylinder (4) verschoben werden kann, um die Kapazität der zweiten Kammer (4a) zu verändern; und
eine Zylinderhalterungseinheit (7), welche die Basis des ersten Kolbens (3), die aus dem ersten Zylinder (2) hervorsteht, und eine Basis des zweiten Kolbens (5) enthält, der aus dem zweiten Zylinder (4) so hervorsteht, dass diese Basen befestigt und entfernt werden können,
**dadurch gekennzeichnet, dass**
der erste und der zweite Zylinder (2, 4) auf einer axialen Linie angeordnet sind, wobei ihre Köpfe (22d, 42d) getrennt miteinander verbunden werden und so angeordnet sind, dass die erste und die zweite Kammer (2a, 4a) mindestens über die erste enge Bohrung (2b) und die zweite enge Bohrung (4b) miteinander kommunizieren; so dass
ein zu verknetendes Material, welches mindestens in die erste Kammer (2a) und die zweite Kammer (4a) eingefüllt wird, durch die Bewegung des ersten und des zweiten Zylinders (2, 4) in einer Richtung entlang der axialen Linie verknetet werden kann.

2. Knetvorrichtung nach Anspruch 1,
**dadurch gekennzeichnet, dass**
das zu verknetende Material ein Zementpulver und eine Härtungsflüssigkeit enthält, welche mit dem Zementpulver reagiert.

3. Knetvorrichtung nach einem der Ansprüche 1 oder 2,
**dadurch gekennzeichnet, dass**
die Einspritzeinheit mindestens an einem Kopf (22d, 42d) des ersten Zylinders (2) und des zweiten Zylinders (4) befestigt werden kann.

4. Knetverfahren, das folgende Schritte umfasst:
Bereitstellung einer Knetvorrichtung, die folgendes enthält: einen ersten Zylinder (2) mit einer ersten Kammer (2a) und einer ersten engen Bohrung (2b), die in dem Kopf (22d) des ersten Zylinders (2) angeordnet ist und mit der ersten Kammer (2a) kommuniziert; sowie einen ersten Kolben (3), welcher in dem ersten Zylinder (2) verschoben werden kann, um dadurch die Kapazität der ersten Kammer (2a) zu verändern; einen zweiten Zylinder (4) mit einer zweiten Kammer (4a) und einer zweiten engen Bohrung (4b), die in dem Kopf (42d) des zweiten Zylinders angeordnet ist und mit der zweiten Kammer (4a) kommuniziert, sowie einen zweiten Kolben (5), der in dem zweiten Zylinder (4) verschoben werden kann, um dadurch die Kapazität der zweiten Kammer (4a) zu verändern, sowie eine Zylinderhalterungseinheit (7);
und das zu verknetende Material mindestens in die erste Kammer (2a) und die zweite Kammer (4a) eingefüllt wird;
und der erste Zylinder (2) und der zweite Zylinder (4) entlang der axialen Linie mit den getrennt verbundenen Köpfen (22d, 42d) so angeordnet wird, dass sie erste und die zweite Kammer (2a, 4a) mindestens mit Hilfe der ersten engen Bohrung (2b) und der zweiten engen Bohrung (4b) miteinander verbunden werden;
Verbindung der Basis des ersten Kolbens (3), die aus dem ersten Zylinder (2) hervorsteht, mit der Basis des zweiten Kolbens (5), welche aus dem zweiten Zylinder (4) hervorsteht, um sie an die Zylinderhalterungseinheit (7) anzuschließen; und
Verschiebung der ersten und zweiten Zylinder (2, 4) in der axialen Richtung gegenüber der Zylinderhalterungseinheit (7), um das Material zu verkneten, das mindestens in die erste Kammer (2) und die zweite Kammer (4) eingefüllt worden ist.

5. Knetverfahren nach Anspruch 4,
**dadurch gekennzeichnet, dass**
das zu verknetende Material ein Zementpulver und eine Härtungsflüssigkeit enthält, welche mit dem Zementpulver reagiert.

6. Knetverfahren nach einem der Ansprüche 4 oder 5,
**dadurch gekennzeichnet, dass**
nachdem der zu verknetende Gegenstand verknetet worden ist, eine Einspritzeinheit mindestens an einem der Köpfe (22d, 42d) des ersten Zylinders (2) und des zweiten Zylinders (4) befestigt wird, und der zu verknetende Gegenstand in das zu injizierende Material eingespritzt wird.

## Revendications

1. Appareil de pétrissage comprenant:
un premier cylindre (2) incluant une première chambre (2a) et un premier trou étroit (2b) prévu à la tête (22d) du premier cylindre (2) et communicant avec la première chambre (2a);
un premier piston (3) déplaçable dans le premier cylindre (2) pour varier ainsi la capacité de la première chambre (2a);
un deuxième cylindre (4) comprenant une deuxième chambre (4a) ainsi qu'un deuxième trou étroit (4b) situé dans la tête (42d) du deuxième cylindre (4) et
communicant avec la deuxième chambre (4a);
un deuxième piston (5) déplaçable dans le deuxième cylindre pour varier ainsi la capacité de la deuxième chambre (4a); et
une unité de support du cylindre (7) portant la base du premier piston (3) s'étendant du premier cylindre (2), ainsi qu'une base du deuxième piston (5) s'étendant du deuxième cylindre (4) de telle sorte que ces bases peuvent être attachées et enlevées;
**caractérisé en ce que**
le premier et le deuxième cylindre (2, 4) sont situés sur une ligne axiale tandisque leur têtes (22d, 42d) sont connectées séparemment de telle sorte que la première et la deuxième chambre (2a, 4a) communiquent entre elles à travers au moins du premier trou étroit (2b) et du deuxième trou étroit (4b), et
**en ce qu'**un objet pétrissable qui peut être inséré dans au moins la première chambre (2a) et la deuxième chambre (4a) peut être pétrissé à l'aide du mouvement des premier et deuxième cylindres (2, 4) dans la direction axiale.

2. Appareil de pétrissage suivant la revendication 1,
**caractérisé en ce que**
l'objet à pétrisser comprend une poudre de cément et un liquide durcissant qui réagit avec la poudre de ciment.

3. Appareil de pétrissage suivant l'une des revendications 1 ou 2,
**caractérisé en ce que**
une unité d'injection peut être fixée à au moins l'une des têtes (22d, 42d) du premier cylindre (2) et du deuxième cylindre (4).

4. Procédé de pétrissage comprenant les étapes suivantes:
mise à disposition d'un appareil de pétrissage comprenant: un premier cylindre (2) avec une première chambre (2a) et un premier trou étroit (2b) prévu dans la tête (22d) du premier cylindre (2) et qui communique avec la première chambre (2a); un premier piston (3) déplaçable dans le premier cylindre pour varier ainsi la capacité de la première chambre (2a); un deuxième cylindre (4) comprenant une deuxième chambre (4a) et un deuxième trou étroit (4b) situé dans la tête (42d) du deuxième cylindre et qui communique avec la deuxième chambre (4a); un deuxième piston (5) déplaçable dans le deuxième cylindre (4) pour varier ainsi la capacité de la deuxième chambre (4a); ainsi qu'une unité de support de cylindre (7);
l'insertion d'un objet pétrissable dans au moins l'une des première chambre (2a) et
deuxième chambre (4a);
disposition d'un premier cylindre (2) et d'un deuxième cylindre (4) sur une ligne axiale et dont les têtes (22d, 42d) ont été jointes séparémment de telle sorte que les première et
deuxième chambres (2a, 4a) sont connectées par l'intermédiaire d'au moins l'un des premier trou étroit (2b) et deuxième trou étroit (4b);
connection de la base du premier piston (3) saillissant du premier cylindre (2) et d'une base du deuxième cylindre (5) saillissant du deuxième cylindre (4) avec l'unité de support du cylindre (7); et
déplacement des premier et deuxième cylindres (2, 4) dans la direction d'une ligne axiale par rapport à l'unité de support du cylindre (7) pour pétrisser l'objet introduit dans au moins l'une des première chambre (2) et deuxième chambre (4).

5. Procédé de pétrissage suivant la revendication 4,
**caractérisé en ce que**
l'objet à pétrisser comprend une poudre de ciment et un liquide durcissant qui réagit avec ladite poudre de ciment.

6. Appareil de pétrissage suivant l'une des revendications 4 ou 5,
**caractérisé en ce que**
après le pétrissage de l'objet à pétrisser une unité d'injection est fixée à au moins l'une des têtes (22d, 42d) des premier cylindre (2) et deuxième cylindre (4) et l'objet à pétrisser est injecté dans l'objet d'injection.
